# EUROPEAN PATENT APPLICATION

(11) **EP 4 733 293 A1**
(43) Date of publication of application: **29.04.2026**
(21) Application number: 24825617.4
(22) Date of filing: 16.05.2024
(51) Int. Cl.: C07C 17/093, C07B 39/00, C07B 53/00, C07C 19/08, C07C 22/00, C07C 22/08, C07C 23/06, C07C 23/08, C07C 23/10, C07C 23/14, C07C 23/16, C07C 23/18, C07C 25/13, C07C 41/22, C07C 41/48, C07C 43/12, C07C 43/313, C07C 67/307, C07C 69/65, C07C 269/06, C07C 271/14

(54) **METHOD FOR PRODUCING ALKYL FLUORIDE COMPOUND, AND METHOD FOR PRODUCING OPTICALLY ACTIVE ALKYL FLUORIDE COMPOUND**

(30) Priority: 22.06.2023 JP 2023102857
(71) Applicant: Mitsubishi Materials Electronic Chemicals Co., Ltd., Akita-shi Akita 010-8585 (JP)
(72) Inventor: NEAGARI WATABE, Yota, Akita-shi, Akita 010-8585 (JP); MATSUMURA, Noriaki, Akita-shi, Akita 010-8585 (JP); KAMIYA, Takeshi, Akita-shi, Akita 010-8585 (JP)
(74) Representative: Hoffmann Eitle
(86) International application number: PCT/JP2024/018211
(87) International publication number: WO 2024/262208

(57) **Abstract**

There is provided a method of efficiently producing an alkyl fluoride compound by directly converting an aliphatic alkylamine widely present in nature into a fluorine substituent, where the method is characterized in that an alkylamine compound having at least one amino group bonded to an alkyl carbon atom is reacted with a deaminative fluorinating agent to generate an alkyl fluoride compound.

## Description

### TECHNICAL FIELD

The present invention relates to a method for producing an alkyl fluoride compound, and a method for producing an optically active alkyl fluoride compound. Priority is claimed on Japanese Patent Application No. 2023-102857, filed June 22, 2023, the content of which is incorporated herein by reference.

### BACKGROUND ART

Organic compounds containing fluorine, such as alkyl fluoride compounds, often exhibit specific properties due to the electronic and steric effects of a fluorine atom. For this reason, alkyl fluoride compounds are used as particularly useful compound in fields such as pharmaceuticals, agricultural chemicals, functional materials, and organic synthetic intermediates. In order to exhibit these properties, it is important to introduce a fluorine group at an appropriate position, and a method for efficiently substituting various functional groups with fluorine groups is desired.

In the related art, alkyl fluoride compounds are generally synthesized from alcohols or alkyl halide compounds, and in particular, a synthesis method using a deoxidative-fluorinating agent has mainly been used.

For example, it is known that deoxidative-fluorinating agents and dehalogenative-fluorinating agents are used in a reaction in which a specific functional group bonded to an alkyl group is substituted with a fluorine group.

There are various methods for synthesizing such alkyl fluoride compounds, and deoxidative fluorination reactions of aliphatic alcohols are generally used (see, for example, Non Patent Document 1). In the method disclosed in Non Patent Document 1, a fluorinating agent reacts with an alcohol, and an oxygen substituent is nucleophilically converted into a fluorine substituent.

Examples of the deoxidative-fluorinating agents that are used in such a method include diethylaminosulfur trifluoride (DAST), bis(2-methoxyethyl)aminosulfur trifluoride (DeoxoFluor), and 4-tert-butyl-2,6-dimethylphenylsulfur trifluoride (Fluolead).

In addition to those described above, for example, reactions in which alkyl fluorides are synthesized by allowing a nucleophilic fluorine source such as tetrabutylammonium fluoride (TBAF) or potassium fluoride to act on alkyl halide compounds or pseudo-alkyl halide compounds such as alkyl sulfonic acid esters, are also known as the reactions in which a specific functional group bonded to an alkyl group is substituted with a fluorine group (see, for example, Non Patent Document 2).

On the other hand, no reaction is known in which a primary alkylamine is directly subjected to deaminative fluorination. Here, amino groups (NH₂) on alkyl groups are structures that are widely seen, in particular in natural products. Despite the abundance of such substituents, only a very limited number of examples of conversion reactions in which primary alkylamines undergo deamination have been reported (see Non Patent Documents 3, 4), and a reaction in which a direct conversion into an alkyl fluoride is carried out is not known.

For example, the Balz-Schiemann reaction is known as a method for synthesizing fluorine compounds by converting an amino group into a fluorine substituent, and this is a method for obtaining aromatic fluorine compounds by thermal decomposition of aromatic diazonium tetrafluoroborates. An example of the reaction of converting an amino group into a fluorine substituent in this way is publicly known for aromatic alkylamines (for example, see Non Patent Document 5); however, a method of directly converting aliphatic alkylamines into a fluorine substituent to synthesize fluorine compounds is not known.

### Citation List

### Patent Document

### [Non-Patent Documents]

[Non Patent Document 1] Ayane Sudo; Junichiro Yamaguchi. Journal of Synthetic Organic Chemistry, Japan, vol. 79, 910-967 (2021).
[Non Patent Document 2] Gabriele Pupo; Veronique Gouverneur. Journal of the American Chemical Society (2022), 144(12), 5200-5213.
[Non Patent Document 3] Kathleen J. Berger; Mark D. Levin. Organic & Biomolecular Chemistry (2020), 19, 11-36.
[Non Patent Document 4] Jiang-Hao Xue; Yin Li; Dong-Hang Tan; Fang-Hai Tu; Yuan Liu; Qingjiang Li; Honggen Wang. iScience (2023), 26(3), 106255.
[Non Patent Document 5] R. Manuel Perez-Garcia; Gaute Gronnevik; Patrick J. Riss. Organic Letters (2021), 23(3), 1011-1015.

### SUMMARY OF THE INVENTION

### Technical Problem

Although such methods of stepwise synthesizing alkyl fluoride compounds as shown in the Non Patent Document 1 to 4 described above, or such methods of subjecting aromatic alkylamines to direct deamination to synthesize alkylaryl fluoride compounds as shown in the Non Patent Document 5 are known, a method of subjecting an aliphatic alkylamine to direct deamination to synthesize alkyl fluoride compounds is not known.

The present invention has been made in consideration of the above-described circumstances, and an object of the present invention is to provide a method of efficiently producing an alkyl fluoride compound by directly converting an aliphatic alkylamine, which is widely present in nature, into a fluorine substituent.

### Solution to Problem

The inventors of the present invention have newly found a synthesis reaction that makes it possible to generate an alkyl fluoride compound by directly substituting an amino group constituting a primary alkylamine compound with a fluorine group by a fluorinating agent. In other words, by focusing on a compound having two reaction points (sulfonyl fluoride groups) in the same molecule, which is represented by a compound represented by FSO₂(CF₂)₃SO₂F, and using such a compound as a deaminative fluorinating agent, an alkyl fluoride compound can be efficiently produced by directly converting an amino group into a fluorine substituent.

In order to solve the above object, a method for producing an alkyl fluoride compound according to an aspect 1 of the present invention is characterized in that an alkylamine compound represented by General Formula (1), which has at least one amino group bonded to an alkyl carbon atom, is reacted with a deaminative fluorinating agent to generate an alkyl fluoride compound represented by General Formula (2).

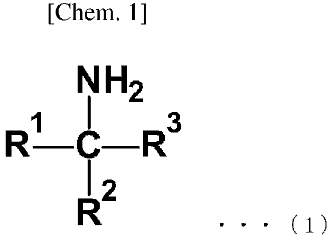

(In General Formula (1), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring. In addition, an amino group (-NH₂) may form a salt by being combined with an acid such as a hydrogen halide or sulfuric acid.)

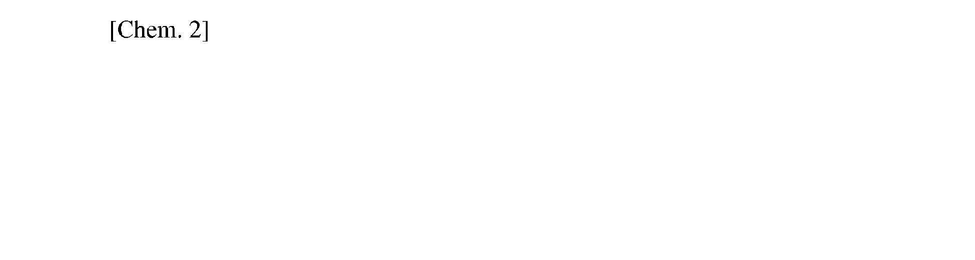

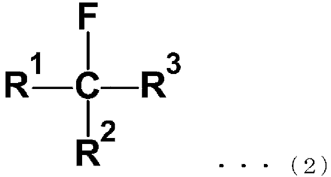

(In General Formula (2), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring).

An aspect 2 according to the present invention is characterized in that in the method for producing an alkyl fluoride compound according to the aspect 1, the deaminative fluorinating agent is a compound represented by General Formula (3).

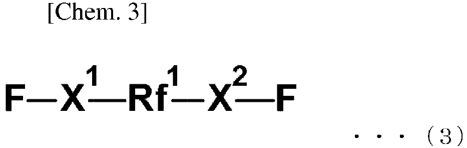

(In General Formula (3), Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or is an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group.)

A method for producing an alkyl fluoride compound according to an aspect 3 of the present invention is characterized by including a first reaction step of reacting the alkylamine compound according to the aspect 1 with the deaminative fluorinating agent according to the aspect 2 to generate an intermediate compound represented by General Formula (4), and a second reaction step of decomposing the intermediate compound to generate the alkyl fluoride compound according to the aspect 1.

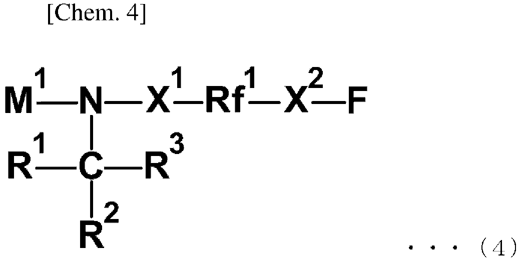

(In General Formula (4), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, R¹, R², and R³ may be bonded to each other to form a 3- to 8-membered carbon ring or a 3- to 8-membered heterocyclic ring having one or more hetero atoms such as oxygen, nitrogen, or sulfur in a ring, and Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group, and M¹ represents hydrogen, a metal, or an organic quaternary ammonium.)

An aspect 4 according to the present invention is characterized in that in the method for producing an alkyl fluoride compound according to the aspect 3, the first reaction step and the second reaction step are allowed to proceed at the same time by reacting the alkylamine compound with the deaminative fluorinating agent in a presence of a basic substance.

An aspect 5 according to the present invention is characterized in that in the method for producing an alkyl fluoride compound according to the aspect 4, a nucleophilic fluorine source material is added together with the alkylamine compound, the deaminative fluorinating agent, and the basic substance.

A method for producing an alkyl fluoride compound according to an aspect 6 of the present invention is characterized in that a compound represented by General Formula (4) is reacted with a deaminative fluorinating agent to generate an alkyl fluoride compound represented by General Formula (2).

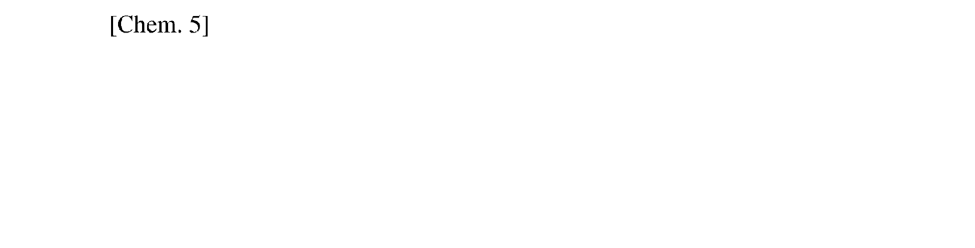

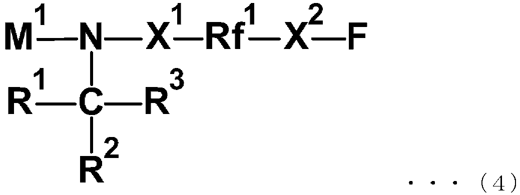

(In General Formula (4), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, R¹, R², and R³ may be bonded to each other to form a 3- to 8-membered carbon ring or a 3- to 8-membered heterocyclic ring having one or more hetero atoms such as oxygen, nitrogen, or sulfur in a ring, and Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group, and M¹ represents hydrogen, a metal, or an organic quaternary ammonium.)

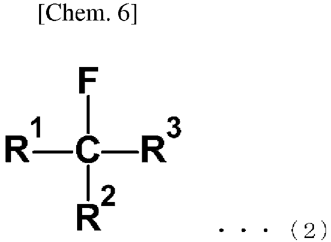

(In General Formula (2), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring).

A method for producing an optically active alkyl fluoride compound according to an aspect 7 of the present invention is characterized in that an optically active alkylamine compound represented by General Formula (5), which has at least one amino group bonded to an alkyl carbon atom, is reacted with a deaminative fluorinating agent to generate an optically active alkyl fluoride compound represented by General Formula (6).

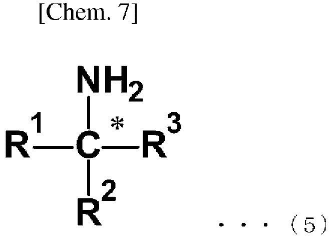

(In General Formula (5), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring. In addition, an amino group (-NH₂) may form a salt by being combined with an acid such as a hydrogen halide or sulfuric acid. C* represents an asymmetric carbon atom.)

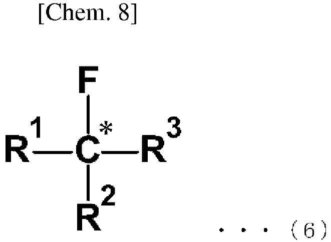

(In General Formula (6), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring. C* represents an asymmetric carbon atom.)

### Advantageous Effects of Invention

According to the present invention, it is possible to provide a method of efficiently producing an alkyl fluoride compound by directly converting an aliphatic alkylamine widely present in nature into a fluorine substituent.

### DESCRIPTION OF EMBODIMENTS

Hereinafter, a method for producing an alkyl fluoride compound according to one embodiment of the present invention and a method for producing an optically active alkyl fluoride compound according to one embodiment of the present invention will be described. It is noted that the embodiments shown below is specifically described to allow the gist of the invention to be better understood and do not limit the present invention unless otherwise specified.

### [Method for producing alkyl fluoride compound]

In the method for producing an alkyl fluoride compound according to the present embodiment, an alkylamine compound represented by General Formula (1), which has at least one amino group bonded to an alkyl carbon atom, is reacted with a deaminative fluorinating agent to generate an alkyl fluoride compound represented by General Formula (2).

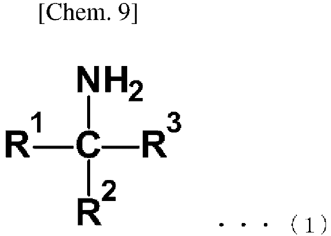

(In General Formula (1), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring. In addition, an amino group (-NH₂) may form a salt by being combined with an acid such as a hydrogen halide or sulfuric acid.)

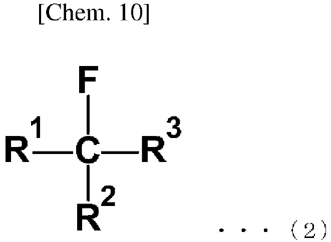

(In General Formula (2), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring).

In addition, the method for producing an alkyl fluoride compound according to the present embodiment may have a configuration in which a first reaction step of reacting the alkylamine compound represented by General Formula (1) with a deaminative fluorinating agent represented by General Formula (3) to generate an intermediate compound represented by General Formula (4), and a second reaction step of decomposing the intermediate compound to generate the alkyl fluoride compound represented by General Formula (2) are provided.

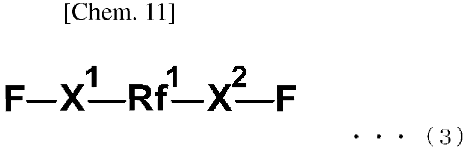

(In General Formula (3), Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or is an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group.)

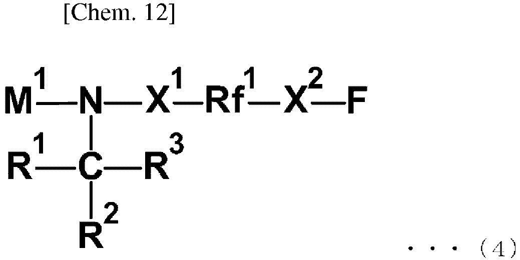

(In General Formula (4), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, R¹, R², and R³ may be bonded to each other to form a 3- to 8-membered carbon ring or a 3- to 8-membered heterocyclic ring having one or more hetero atoms such as oxygen, nitrogen, or sulfur in a ring, and Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group, and M¹ represents hydrogen, a metal, or an organic quaternary ammonium.)

In General Formulae (1) and (2), specific examples of the alkyl group which may have a substituent or an unsaturated bond include an alkyl group in which any carbon atoms of the alkyl group have undergone any number of substitutions with an halogen atom of fluorine, chlorine, bromine, or iodine, an alkoxy group such as a methoxy group, an ethoxy group, or a propoxy group, a haloalkoxy group such as a fluoromethoxy group, a chloromethoxy group, or a bromomethoxy group, a dialkylamino group such as a dimethylamino group, a diethylamino group, or a dipropylamino group, an alkylthio group such as a methylthio group, an ethylthio group, or a propylthio group, an ester group such as a cyano group, a nitro group, a formyl group, a methyl ester group, an ethyl ester group, or a propyl ester group, an unsaturated group such as alkenyl group or an alkynyl group, an aromatic ring group such as a phenyl group or a naphthyl group, an aryloxy group such as a phenoxy group or a naphthoxy group, a protected form of a hydroxy group, a protected form of an amino group, a protected form of a thiol group, a protected form of a carboxyl group, or the like, in any combination. It is noted that in the present specification, the phrase "may have" means "has (have) or may not have". In addition, the phrase "may form" means "form(s) or may not form".

In General Formulae (1) and (2), specific examples of the aryl group which may have a substituent or an unsaturated bond include an aryl group in which an halogen atom of fluorine, chlorine, bromine, or iodine, an alkoxy group such as a methoxy group, an ethoxy group, or a propoxy group, a haloalkoxy group such as a fluoromethoxy group, a chloromethoxy group, or a bromomethoxy group, a dialkylamino group such as a dimethylamino group, a diethylamino group, or a dipropylamino group, an alkylthio group such as a methylthio group, an ethylthio group, or a propylthio group, an ester group such as a cyano group, a nitro group, a formyl group, a methyl ester group, an ethyl ester group, or a propyl ester group, an unsaturated group such as alkenyl group or an alkynyl group, an aromatic ring group such as a phenyl group or a naphthyl group, an aryloxy group such as a phenoxy group or a naphthoxy group, a protected form of a hydroxy group, a protected form of an amino group, a protected form of a thiol group, a protected form of a carboxyl group, or the like has undergone, on any carbon atom of an aryl group, any number of substitutions in any combination.

In General Formulae (1) and (2), specific examples of the carbonyl group having an alkyl group which may have a substituent or an unsaturated bond include a carbonyl group having an alkyl group in which an halogen atom of fluorine, chlorine, bromine, or iodine, an alkoxy group such as a methoxy group, an ethoxy group, or a propoxy group, a haloalkoxy group such as a fluoromethoxy group, a chloromethoxy group, or a bromomethoxy group, a dialkylamino group such as a dimethylamino group, a diethylamino group, or a dipropylamino group, an alkylthio group such as a methylthio group, an ethylthio group, or a propylthio group, an ester group such as a cyano group, a nitro group, a formyl group, a methyl ester group, an ethyl ester group, or a propyl ester group, an unsaturated group such as alkenyl group or an alkynyl group, an aromatic ring group such as a phenyl group or a naphthyl group, an aryloxy group such as a phenoxy group or a naphthoxy group, a protected form of a hydroxy group, a protected form of an amino group, a protected form of a thiol group, a protected form of a carboxyl group, or the like has undergone, on any carbon atom of an alkyl group, any number of substitutions in any combination.

In General Formulae (1) and (2), specific examples of the carbonyl group having an aryl group which may have a substituent or an unsaturated bond include a carbonyl group having an aryl group in which an halogen atom of fluorine, chlorine, bromine, or iodine, an alkoxy group such as a methoxy group, an ethoxy group, or a propoxy group, a haloalkoxy group such as a fluoromethoxy group, a chloromethoxy group, or a bromomethoxy group, a dialkylamino group such as a dimethylamino group, a diethylamino group, or a dipropylamino group, an alkylthio group such as a methylthio group, an ethylthio group, or a propylthio group, an ester group such as a cyano group, a nitro group, a formyl group, a methyl ester group, an ethyl ester group, or a propyl ester group, an unsaturated group such as alkenyl group or an alkynyl group, an aromatic ring group such as a phenyl group or a naphthyl group, an aryloxy group such as a phenoxy group or a naphthoxy group, a protected form of a hydroxy group, a protected form of an amino group, a protected form of a thiol group, a protected form of a carboxyl group, or the like has undergone, on any carbon atom of an aryl group, any number of substitutions in any combination.

In General Formulae (3) and (4), specific examples of the alkyl group allowed to have a linear, branched, or cyclic structure having 1 to 10 carbon atoms, which may have 1 to 5 oxygen atoms having bondability to an ether or 1 to 5 unsaturated bonds between carbon atoms, or the aryl group having 6 to 14 carbon atoms, which may have a substituent, where the alkyl group or the aryl group is an fluoroalkyl group in which one to all hydrogen atoms on a carbon atom are substituted with fluorine, include a difluoromethylene group, a tetrafluoroethylene group, a trifluoromethylfluoromethyl group, a hexafluoropropylene group, an octafluorobutylene group, and a - CF(CF₃)OCF₂CF₂OCF(CF₃)-group.

FSO₂(CF₂)₃SO₂F is most preferable for the substituents Rf¹, X¹, X² of the deaminative fluorinating agent represented by General Formula (3); however, the reaction also proceeds in FSO₂(CF₂)₂SO₂F which has a different chain length, or FSO₂(CF₂)₃COF which is a compound in which a sulfonyl group is substituted with a carbonyl group. However, the reaction does not proceed even in a case where FSO₂Rf, which is not a difunctional type in which fluorine functional groups are present at both terminals, is used.

In addition, the first reaction step and the second reaction step can be allowed to proceed at the same time by reacting the above-described alkylamine compound with the above-described deaminative fluorinating agent in the presence of a basic substance. As a result, the alkyl fluoride compound can be produced more efficiently.

Specific examples of the basic substance that is used to allow the first reaction step and the second reaction step proceed at the same time as described above include trimethylamine, triethylamine, diisopropylamine, tripropylamine, pyridine, 4-dimethylaminopyridine (DMAP), diazabicycloundecene (DBU), 1,5,7-triazabicyclo[4.4.0]dec-5-ene (TBD), diazabicyclononene (DBN), 7-methyl-1,5,7-triazabicyclo[4.4.0]dec-5-ene pyridine (MTBD), tert-butylimino-tris(dimethylamino)phosphorane, tert-butylimino-tri(pyrrolidino)phosphorane (BTPP), and 2-tert-butyl-1,1,3,3-tetramethylguanidine (BTMG). Among them, diazabicycloundecene (DBU) is particularly preferable.

A using amount of the basic substance with respect to the alkylamine compound represented by General Formula (1) is in a range of 0.5 or more and 10 or less in a molar ratio, and preferably in a range of 1.5 or less and 3.0 or less with respect to one part of the reactive amino group (NH₂₋). In a case where the using amount of the basic substance is less than 0.5 in terms of the molar ratio, the amount of the alkyl fluoride compound generated is lower than the reaction equivalent. In addition, in a case where the using amount of the basic substance is more than 10 in terms of the molar ratio, there is a concern that the generated alkyl fluoride compound may decompose.

In addition, in order to allow the first reaction step and the second reaction step to proceed at the same time as described above, a nucleophilic fluorine source material can be added in addition to the alkylamine compound, the deaminative fluorinating agent, and the basic substance.

Specific examples of the nucleophilic fluorine source material described above include sodium fluoride, potassium fluoride, cesium fluoride, silver fluoride, a triethylamine hydrogen trifluoride salt, hydrogen fluoride pyridine, tetramethylammonium fluoride, tetrabutylammonium fluoride (TBAF), and tetrabutylammonium difluorotriphenylsilicate (TBAT). Among them, tetrabutylammonium difluorotriphenylsilicate (TBAT) is particularly preferable.

A using amount of the nucleophilic fluorine source material with respect to the alkylamine compound represented by General Formula (1) is in a range of 0 to 10 in terms of the molar ratio and preferably in a range of 0.3 to 1.0 in terms of the molar ratio with respect to one part of the reactive amino group (NH₂₋). The first reaction step and the second reaction step proceed without a particular addition of the nucleophilic fluorine source material; however, the amount (yield) of the alkyl fluoride compound generated is reduced as compared with a case where the nucleophilic fluorine source material is added. In addition, in a case where the using amount of the nucleophilic fluorine source material is more than 10 in terms of the molar ratio, no further increase in the amount of the alkyl fluoride compound generated is observed, and thus only the cost of addition of the nucleophilic fluorine source material increases.

In the first reaction step or the second reaction step, an addition ratio of the deaminative fluorinating agent, the basic substance, and the nucleophilic fluorine source material to the alkylamine compound is particularly preferably around 2.0:2.0:0.5 (eq); however, the addition ratio is not limited thereto.

A aprotic solvent can be used as a solvent for dissolving the alkylamine compound that is used in the first reaction step or the second reaction step. Specific examples of the aprotic solvent include acetonitrile, propionitrile, dimethylformamide, dimethylacetamide, methylformamide, N-methylpyrrolidone, diethyl ether, diisopropyl ether, tert-butyl methyl ether, cyclopentyl methyl ether, tetrahydrofuran, 1,2-dimethoxyethane, 1,3-dioxane, 1,4-dioxane, diglyme, triglyme, dimethyl sulfoxide, sulfolane, pentane, hexane, heptane, octane, cyclohexane, benzene, toluene, xylene, and ethyl benzene. Among them, toluene is particularly preferable as the solvent. In addition, a plurality of kinds of solvents can also be mixed and used.

Both the reaction temperatures of the first reaction step and the second reaction step are preferably in a range of equal to or higher than 0°C and equal to or lower than the boiling point of the solvent used for dissolving the alkylamine compound. In a case where the reaction temperature is less than 0°C, the progression of the reaction is slowed down. In addition, it is preferable that in the first reaction step or the second reaction step, the reaction is carried out under a condition that the solvent is refluxed.

The reaction time of the first reaction step and the second reaction step is preferably in a range of 1 hour or more and 3 hours or less. However, even in a case where the reaction time is about 30 minutes, 50% or more of the reaction of the total reaction does not proceed, and thus the reaction temperature may be less than 1 hour, depending on the temperature condition.

According to such a method for producing an alkyl fluoride compound according to the present embodiment as described above, the alkyl fluoride compound can be efficiently produced by using a compound having two reaction points in the same molecule as a deaminative fluorinating agent in order to directly substitute an amino group constituting the primary alkylamine compound, which could not be converted with the fluorinating agent in the related art, with a fluorine atom. As a result, it is possible to realize the synthesis of the fluorine compound, the synthesis of which has been difficult in the related art, or the simplification of the synthesis process. In addition, by using, as a raw material for synthesis, the primary alkylamine that is abundant as a natural product, it is possible to efficiently produce the alkyl fluoride compound at a low cost.

### [Method for producing optically active alkyl fluoride compound]

In the method for producing an optically active alkyl fluoride compound according to the present embodiment, an alkylamine compound represented by General Formula (5), which has at least one amino group bonded to an alkyl carbon atom, is reacted with a deaminative fluorinating agent to generate an alkyl fluoride compound represented by General Formula (6).

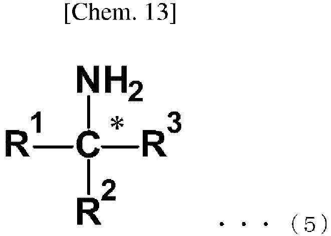

(In General Formula (1), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring. In addition, an amino group (-NH₂) may form a salt by being combined with an acid such as a hydrogen halide or sulfuric acid. C* represents an asymmetric carbon atom.)

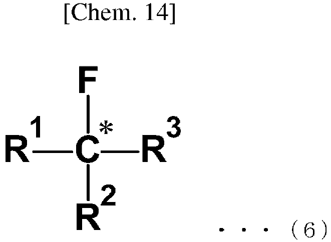

(In General Formula (2), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring. C* represents an asymmetric carbon atom.)

The optically active alkyl fluoride compound is an (optical active) alkyl fluoride compound that is capable of rotating a polarizing surface, and it has optical activity. In a molecular structure of a substance exhibiting optical activity, there is no rotatory reflection axis (symmetric center, mirror surface, and rotational axis) which is a symmetrical element of the second type, and a structure that serves as an antipode is present. It needs to be a chiral compound in order to allow an optical isomer to be present, and a compound having an asymmetric carbon atom in the molecule is often a chiral compound.

In the method for producing an optically active alkyl fluoride compound according to the present embodiment, for specific examples of the alkyl group which may have a substituent or an unsaturated bond in General Formulae (5) and (6), specific examples of the aryl group which may have a substituent or an unsaturated bond, specific examples of the carbonyl group having an alkyl group which have a substituent or an unsaturated bond, and specific examples of the carbonyl group having an aryl group which have a substituent or an unsaturated bond, the same ones as the respective specific examples according to General Formula (1) and (2) in the above-described method for producing an alkyl fluoride compound can be used.

In addition, in the method for producing an optically active alkyl fluoride compound according to the present embodiment, for specific examples of the deaminative fluorinating agent, the basic substance, the nucleophilic fluorine source material, and the solvent, the same ones as the respective specific examples according to the above-described method for producing an alkyl fluoride compound can be also used.

Further, In the method for producing an optically active alkyl fluoride compound according to the present embodiment, reaction conditions (a molar ratio between constitutional materials of a reaction, a range of reaction temperature, and a range of reaction time) the same conditions as those for the respective specific examples according to the above-described method for producing an alkyl fluoride compound can be also used.

As described above, the embodiments according to the present invention have been described. However, such embodiments are presented as examples and thus are not intended to limit the scope of the invention. Such embodiments can be carried out in various other forms, and various omissions, replacements, and changes can be made to the extent that they do not deviate from the gist of the invention. These embodiments and the modification thereof are included in the scope and gist of the invention and also included in the scope of the invention described in the present patent claims and the scope of its equivalents.

### [Examples]

Hereinafter, specific synthesis examples according to the present invention are listed; however, these synthesis examples are examples, and the present invention is not limited thereto.

### [Example 1 of present invention]

A primary alkylamine compound represented by Formula (7) was used to synthesize an alkyl fluoride compound represented by Formula (8).

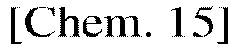

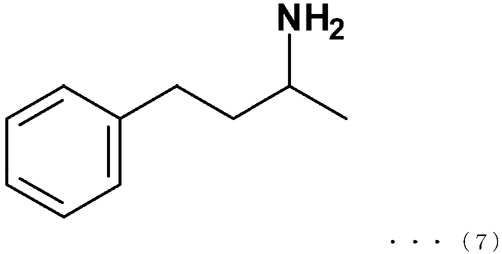

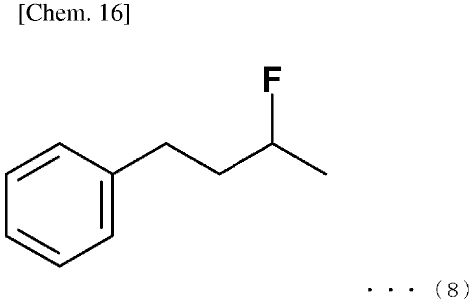

In a nitrogen atmosphere, 3-amino-1-phenylbutane (74.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane/ethyl acetate = 100/1), and the solvent was distilled off under reduced pressure to obtain an alkyl fluoride compound represented by Formula (8) in a yield of 53%.

Then, by a ¹⁹F-NMR analysis, it was confirmed that the obtained product is the alkyl fluoride compound represented by Formula (8).

¹⁹F-NMR (heavy chloroform) analysis: δ of -174.04 to -174.36 (m, 1F).

### [Example 2 of present invention]

A primary alkylamine compound represented by Formula (9) was used to synthesize an alkyl fluoride compound represented by Formula (10).

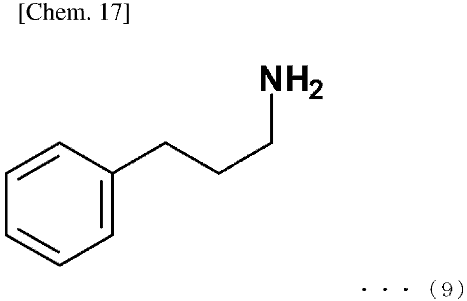

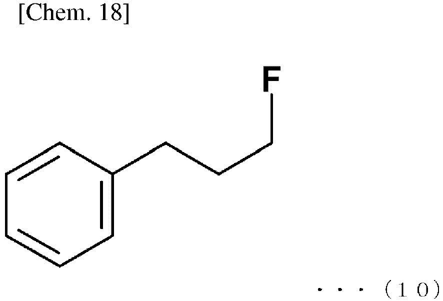

In a nitrogen atmosphere, 3-phenylpropylamine (67.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane/ethyl acetate = 100/1), and the solvent was distilled off under reduced pressure to obtain an alkyl fluoride compound represented by Formula (10) in a yield of 35%.

Then, by a ¹⁹F-NMR analysis, it was confirmed that the obtained product is the alkyl fluoride compound represented by Formula (10).

¹⁹F-NMR (heavy chloroform) analysis: δ of -219.94 to -220.32 (m, 1F).

### [Example 3 of present invention]

A primary alkylamine compound represented by Formula (11) was used to synthesize an alkyl fluoride compound represented by Formula (12).

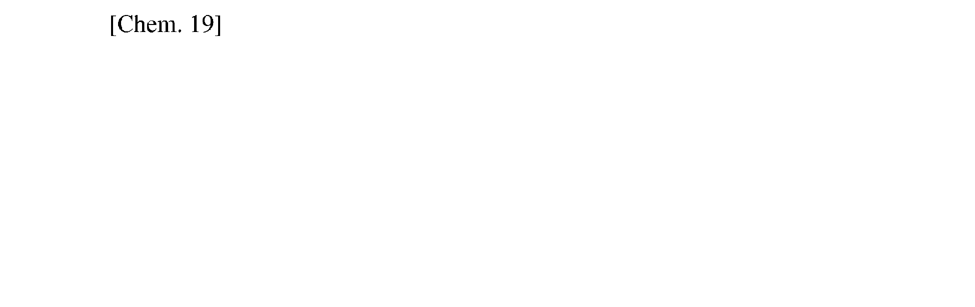

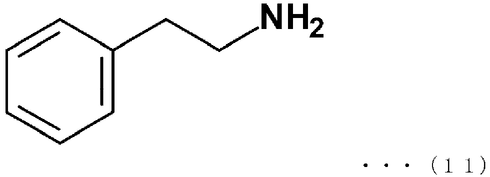

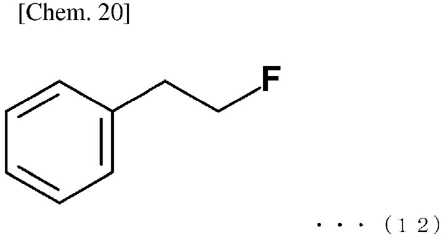

In a nitrogen atmosphere, phenethylamine (60.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane/ethyl acetate = 100/1), and the solvent was distilled off under reduced pressure to obtain an alkyl fluoride compound represented by Formula (12) in a yield of 31%.

Then, by a ¹⁹F-NMR analysis, it was confirmed that the obtained product is the alkyl fluoride compound represented by Formula (12).

¹⁹F-NMR (heavy chloroform) analysis: δ of -215.02 to 215.39 (m, 1F).

### [Example 4 of present invention]

A primary alkylamine compound represented by Formula (13) was used to synthesize an alkyl fluoride compound represented by Formula (14).

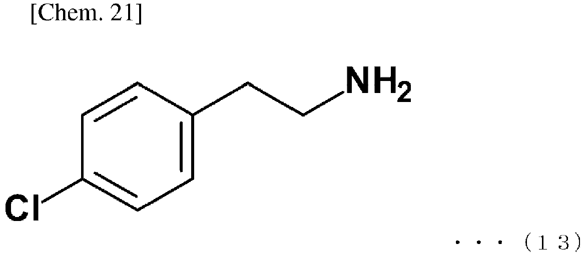

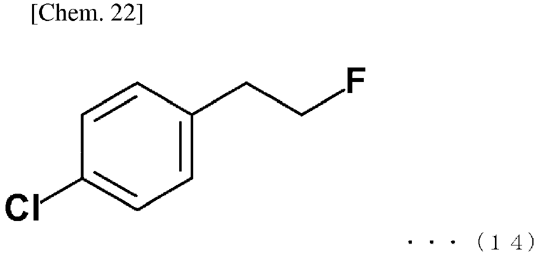

In a nitrogen atmosphere, 2-(4-chlorophenyl)ethylamine (77.8 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane/ethyl acetate = 100/1), and the solvent was distilled off under reduced pressure to obtain an alkyl fluoride compound represented by Formula (14) in a yield of 44%.

Then, by a ¹⁹F-NMR analysis, it was confirmed that the obtained product is the alkyl fluoride compound represented by Formula (14).

¹⁹F-NMR (heavy chloroform) analysis: δ of -215.87 to -216.24 (m, 1F).

### [Example 5 of present invention]

A primary alkylamine compound represented by Formula (15) was used to synthesize an alkyl fluoride compound represented by Formula (16).

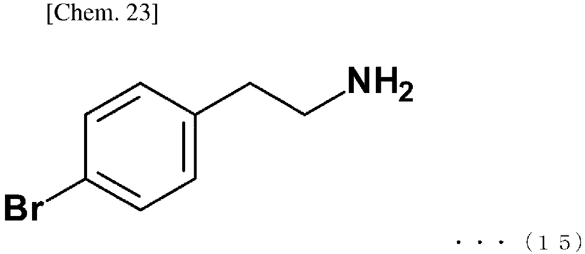

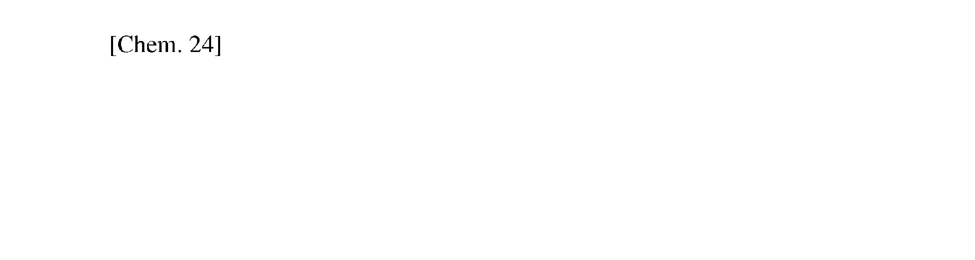

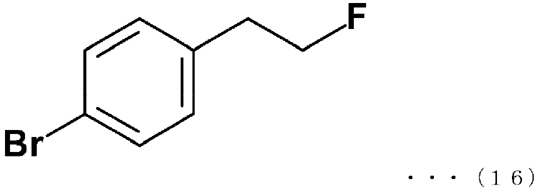

In a nitrogen atmosphere, 2-(4-bromophenyl)ethylamine (100.0 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane to hexane/ethyl acetate = 20/1), and the solvent was distilled off under reduced pressure to obtain an alkyl fluoride compound represented by Formula (16) in a yield of 32%.

Then, by a ¹⁹F-NMR analysis, it was confirmed that the obtained product is the alkyl fluoride compound represented by Formula (16).

¹⁹F-NMR (heavy chloroform) analysis: δ of -215.88 to -216.25 (m, 1F).

### [Example 6 of present invention]

A primary alkylamine compound represented by Formula (17) was used to synthesize an alkyl fluoride compound represented by Formula (18).

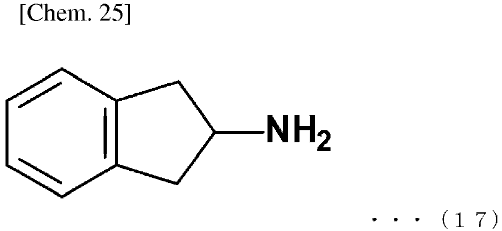

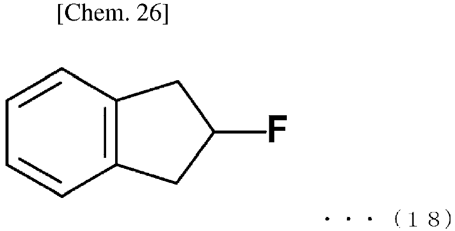

**In** a nitrogen atmosphere, 2-aminoindane (66.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane to hexane/ethyl acetate = 100/1), and the solvent was distilled off under reduced pressure to obtain an alkyl fluoride compound represented by Formula (18) in a yield of 28%.

Then, by a ¹⁹F-NMR analysis, it was confirmed that the obtained product is the alkyl fluoride compound represented by Formula (18).

¹⁹F-NMR (heavy chloroform) analysis: δ of -173.62 to -174.09 (m, 1F).

### [Example 7 of present invention]

A primary alkylamine compound represented by Formula (19) was used to synthesize an alkyl fluoride compound represented by Formula (20).

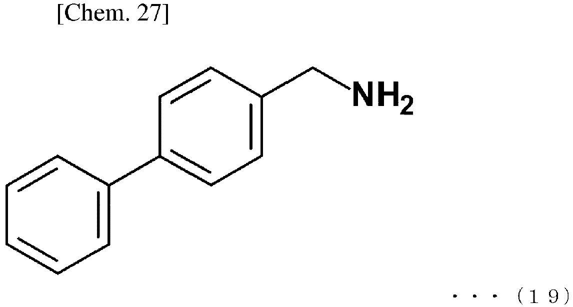

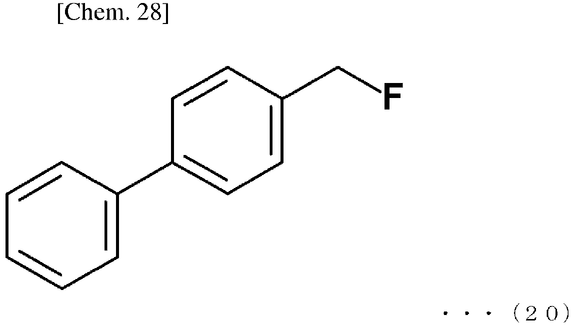

In a nitrogen atmosphere, 4-phenylbenzylamine (91.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane/ethyl acetate = 20/1), and the solvent was distilled off under reduced pressure to obtain an alkyl fluoride compound represented by Formula (20) in a yield of 14%.

Then, by a ¹⁹F-NMR analysis, it was confirmed that the obtained product is the alkyl fluoride compound represented by Formula (20).

¹⁹F-NMR (heavy chloroform) analysis: δ of -206.29 (t, 1F).

### [Example 8 of present invention]

A primary alkylamine compound represented by Formula (21) was used to synthesize an alkyl fluoride compound represented by Formula (22).

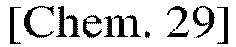

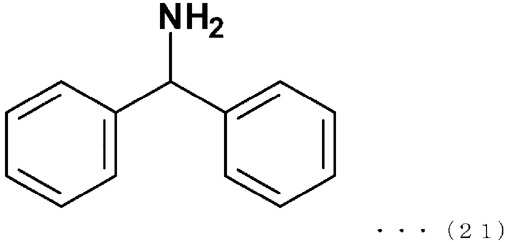

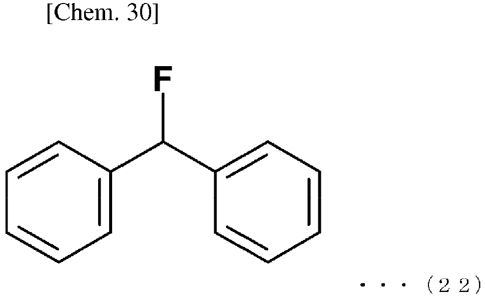

In a nitrogen atmosphere, benzhydrylamine (91.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane to hexane/ethyl acetate = 20/1), and the solvent was distilled off under reduced pressure to obtain an alkyl fluoride compound represented by Formula (22) in a yield of 55%.

Then, by a ¹⁹F-NMR analysis, it was confirmed that the obtained product is the alkyl fluoride compound represented by Formula (22).

¹⁹F-NMR (heavy chloroform) analysis: δ of -166.76 (d, 1F).

### [Example 9 of present invention]

A primary alkylamine compound represented by Formula (23) was used to synthesize an alkyl fluoride compound represented by Formula (24).

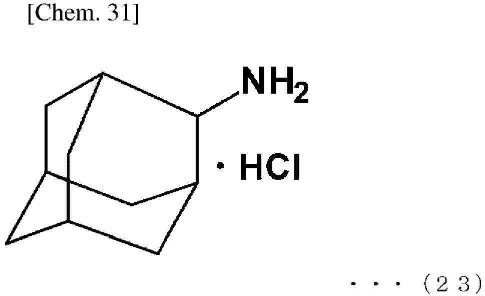

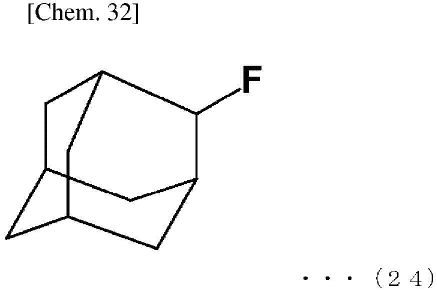

In a nitrogen atmosphere, 2-adamantanamine hydrochloride (93.8 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (228.4 mg, 1.5 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane), and the solvent was distilled off under reduced pressure to obtain an alkyl fluoride compound represented by Formula (24) in a yield of 12%.

Then, by a ¹⁹F-NMR analysis, it was confirmed that the obtained product is the alkyl fluoride compound represented by Formula (24).

¹⁹F-NMR (heavy chloroform) analysis: δ of -173.81 to -173.97 (m, 1F).

### [Example 10 of present invention]

A primary alkylamine compound represented by Formula (25) was used to synthesize an alkyl fluoride compound represented by Formula (26).

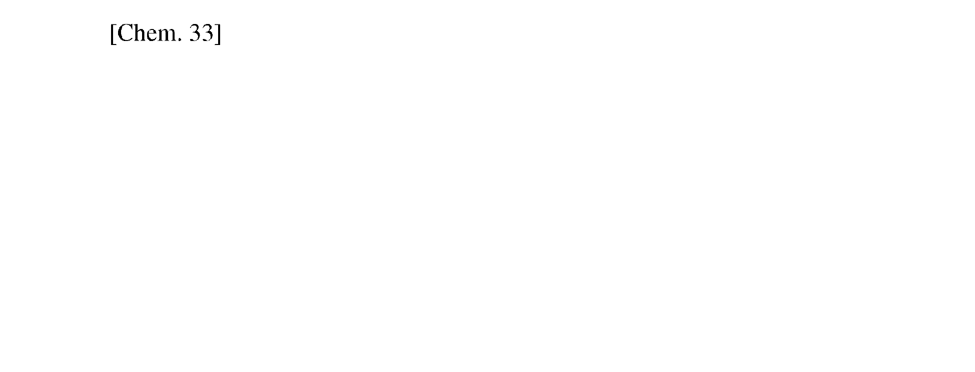

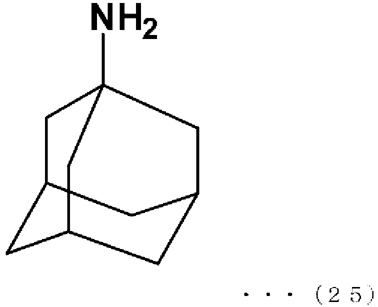

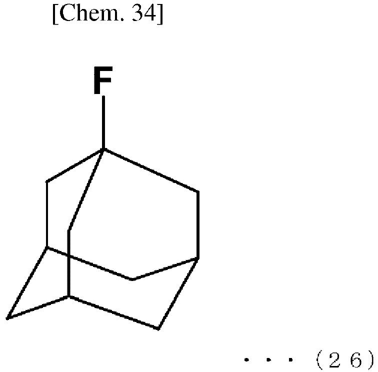

In a nitrogen atmosphere, 1-adamantanamine (75.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane), and the solvent was distilled off under reduced pressure to obtain an alkyl fluoride compound represented by Formula (26) in a yield of 20%.

Then, by a ¹⁹F-NMR analysis, it was confirmed that the obtained product is the alkyl fluoride compound represented by Formula (26).

¹⁹F-NMR (heavy chloroform) analysis: δ of -128.50 to -128.52 (m, 1F).

### [Example 11 of present invention]

A primary alkylamine compound represented by Formula (27) was used to synthesize an alkyl fluoride compound represented by Formula (28).

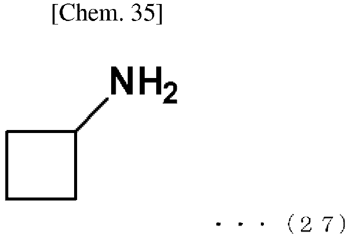

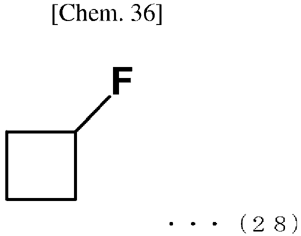

In a nitrogen atmosphere, cyclobutylamine (35.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 40°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (28) was 12%.

Then, by a ¹⁹F-NMR analysis, it was confirmed that the obtained product is the alkyl fluoride compound represented by Formula (28).

¹⁹F-NMR (heavy chloroform) analysis: δ of -159.39 to -159.71 (m, 1F).

### [Example 12 of present invention]

A primary alkylamine compound represented by Formula (29) was used to synthesize an alkyl fluoride compound represented by Formula (30).

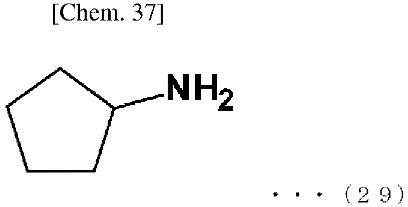

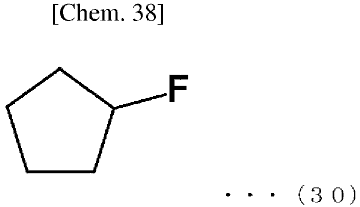

In a nitrogen atmosphere, cyclopentylamine (42.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 40°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (30) was 41%.

Then, by a ¹⁹F-NMR analysis, it was confirmed that the obtained product is the alkyl fluoride compound represented by Formula (30).

¹⁹F-NMR (heavy chloroform) analysis: δ of -169.42 to -169.88 (m, 1F).

### [Example 13 of present invention]

A primary alkylamine compound represented by Formula (31) was used to synthesize an alkyl fluoride compound represented by Formula (32).

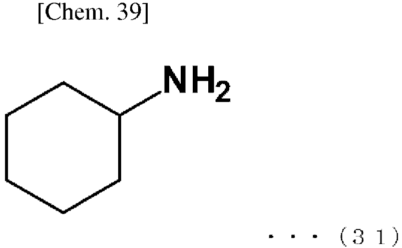

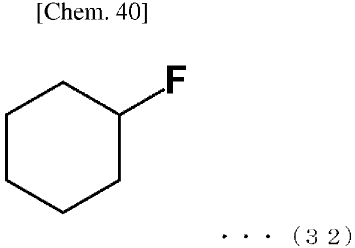

In a nitrogen atmosphere, cyclohexylamine (49.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 40°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (32) was 2%.

Then, by a ¹⁹F-NMR analysis, it was confirmed that the obtained product is the alkyl fluoride compound represented by Formula (32).

¹⁹F-NMR (heavy chloroform) analysis: δ of -171.97 to -172.30 (m, 1F).

### [Example 14 of present invention]

A primary alkylamine compound represented by Formula (33) was used to synthesize an alkyl fluoride compound represented by Formula (34).

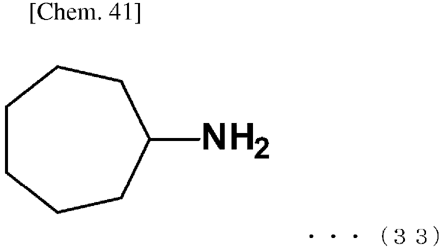

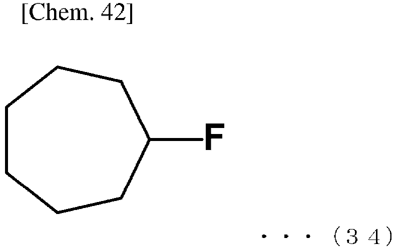

In a nitrogen atmosphere, cycloheptylamine (56.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to benzene (10 mL), and heating was carried out to 80°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (34) was 51%.

Then, by a ¹⁹F-NMR analysis, it was confirmed that the obtained product is the alkyl fluoride compound represented by Formula (34).

¹⁹F-NMR (heavy chloroform) analysis: δ of -163.25 to -163.57 (m, 1F).

### [Example 15 of present invention]

A primary alkylamine compound represented by Formula (35) was used to synthesize an alkyl fluoride compound represented by Formula (36).

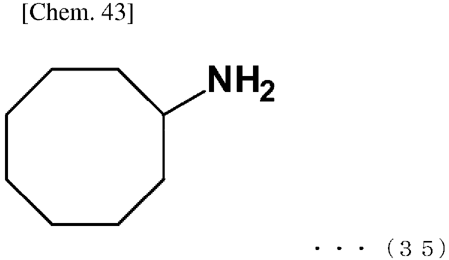

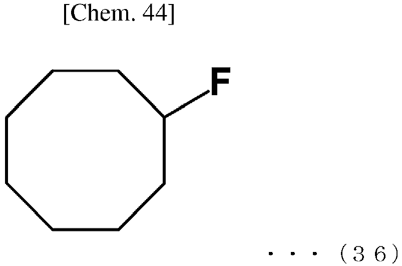

In a nitrogen atmosphere, cyclooctylamine (63.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to benzene (10 mL), and heating was carried out to 80°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (36) was 21%.

Then, by a ¹⁹F-NMR analysis, it was confirmed that the obtained product is the alkyl fluoride compound represented by Formula (36).

¹⁹F-NMR (heavy chloroform) analysis: δ of -158.27 to -158.91 (m, 1F).

### [Example 16 of present invention]

A primary alkylamine compound represented by Formula (37) was used to synthesize an alkyl fluoride compound represented by Formula (38).

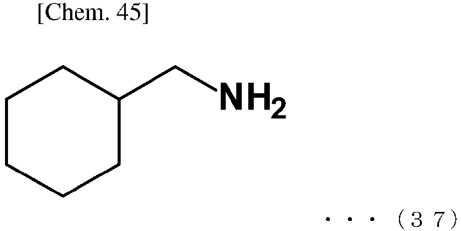

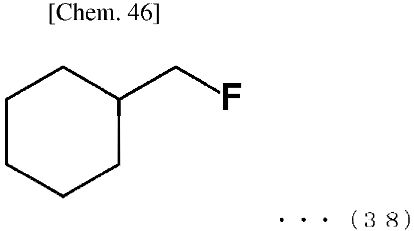

In a nitrogen atmosphere, cyclohexane methylamine (56.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to benzene (10 mL), and heating was carried out to 80°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (38) was 23%.

Then, by a ¹⁹F-NMR analysis, it was confirmed that the obtained product is the alkyl fluoride compound represented by Formula (38).

¹⁹F-NMR (heavy chloroform) analysis: δ of -221.92 to -222.22 (m, 1F).

### [Example 17 of present invention]

A primary alkylamine compound represented by Formula (39) was used to synthesize an alkyl fluoride compound represented by Formula (40).

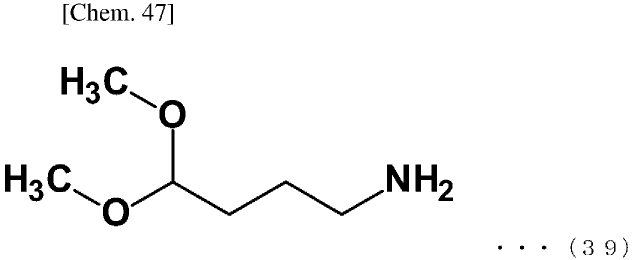

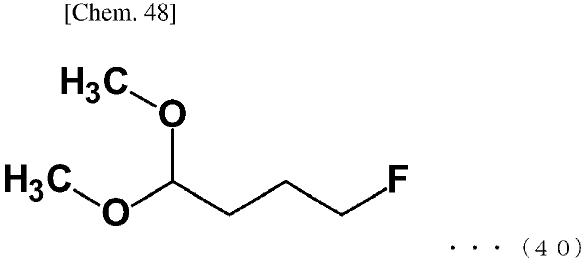

In a nitrogen atmosphere, 4,4-dimethoxybutylamine (66.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (40) was 32%.

Then, by a ¹⁹F-NMR analysis, it was confirmed that the obtained product is the alkyl fluoride compound represented by Formula (40).

¹⁹F-NMR (heavy chloroform) analysis: δ of -218.18 to -218.57 (m, 1F).

### [Example 18 of present invention]

A primary alkylamine compound represented by Formula (41) was used to synthesize an alkyl fluoride compound represented by Formula (42).

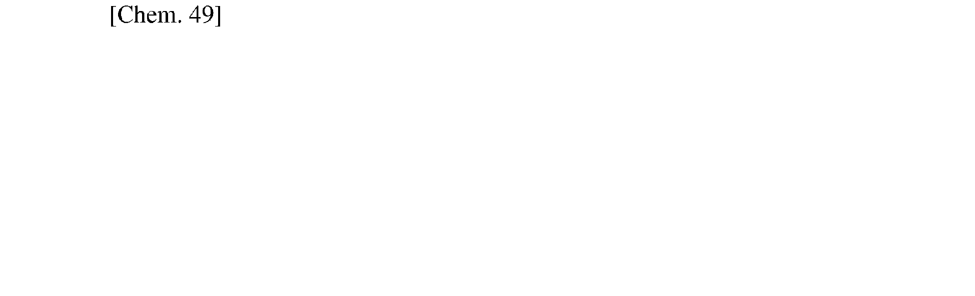

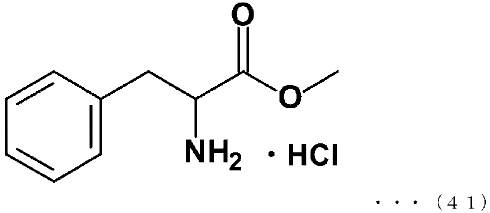

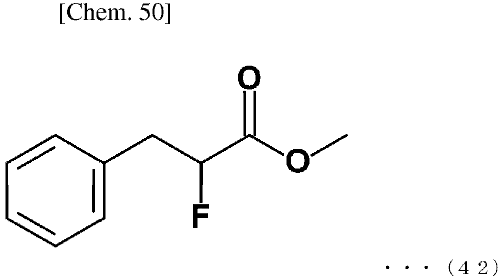

In a nitrogen atmosphere, DL-phenylalanine methyl ester hydrochloride (107.8 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane/ethyl acetate = 10/1), and the solvent was distilled off under reduced pressure to obtain an alkyl fluoride compound represented by Formula (42) in a yield of 3%.

Then, by a ¹⁹F-NMR analysis, it was confirmed that the obtained product is the alkyl fluoride compound represented by Formula (42).

¹⁹F-NMR (heavy chloroform) analysis: δ of -189.97 to -190.24 (m, 1F).

### [Example 19 of present invention]

A primary alkylamine compound represented by Formula (43) was used to synthesize an alkyl fluoride compound represented by Formula (44).

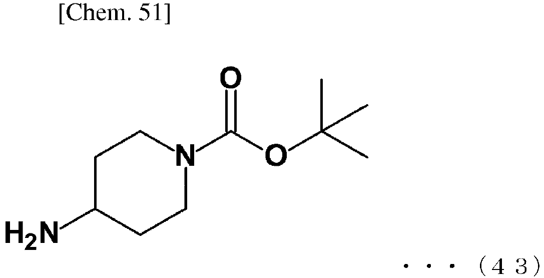

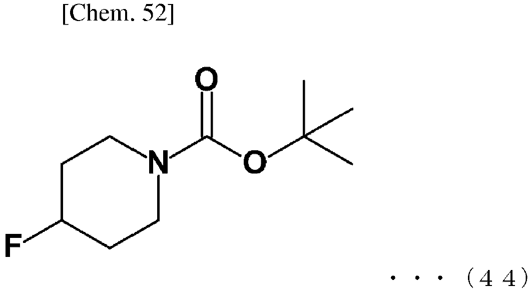

In a nitrogen atmosphere, 1-Boc-4-piperidine amine (100.1 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane), and the solvent was distilled off under reduced pressure to obtain an alkyl fluoride compound represented by Formula (44) in a yield of 33%.

Then, by a ¹⁹F-NMR analysis, it was confirmed that the obtained product is the alkyl fluoride compound represented by Formula (44).

¹⁹F-NMR (heavy chloroform) analysis: δ of -181.99 to -182.33 (m, 1F).

### [Example 20 of present invention]

A primary alkylamine compound represented by Formula (45) was used to synthesize an alkyl fluoride compound represented by Formula (46).

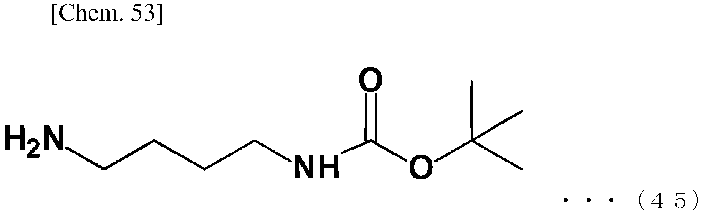

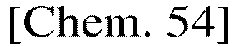

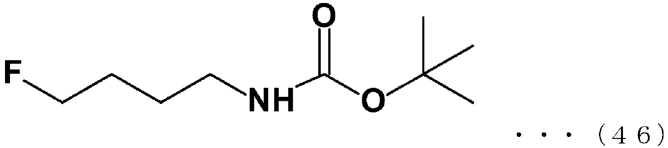

In a nitrogen atmosphere, N-(tert-butoxycarbonyl)-1,4-diaminobutane (94.1 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane), and the solvent was distilled off under reduced pressure to obtain an alkyl fluoride compound represented by Formula (46) in a yield of 16%.

Then, by a ¹⁹F-NMR analysis, it was confirmed that the obtained product is the alkyl fluoride compound represented by Formula (46).

¹⁹F-NMR (heavy chloroform) analysis: δ of -218.16 to -218.55 (m, 1F).

### [Example 21 of present invention]

A primary alkylamine compound represented by Formula (47) was used to synthesize an alkyl fluoride compound represented by Formula (48).

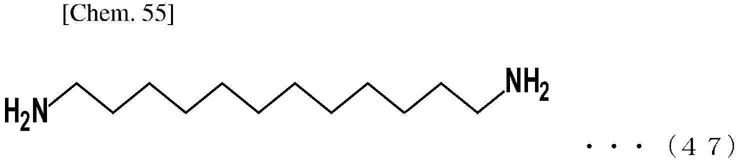

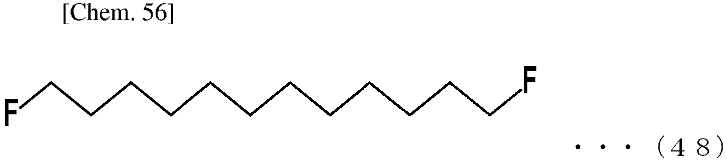

In a nitrogen atmosphere, 1,12-diaminodecane (100.2 mg, 0.50 mmol), 1,1,2,2,3,3-hexafluoropropane-1,3-disulfonylfluoride (632.3 mg, 2.0 mmol), and tetrabutylammonium difluorotriphenylsilicate (TBAT) (270.0 mg, 0.50 mmol) were added to toluene (10 mL), and heating was carried out to 110°C. Diazabicycloundecene (DBU) (304.5 mg, 2.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane to hexane/ethyl acetate = 20/1), and the solvent was distilled off under reduced pressure to obtain an alkyl fluoride compound represented by Formula (48) in a yield of 21%.

Then, by a ¹⁹F-NMR analysis, it was confirmed that the obtained product is the alkyl fluoride compound represented by Formula (48).

¹⁹F-NMR (heavy chloroform) analysis: δ of -217.88 to -218.27 (m, 2F).

### [Example 22 of present invention]

A primary alkylamine compound represented by Formula (49) was used to synthesize an alkyl fluoride compound represented by Formula (50).

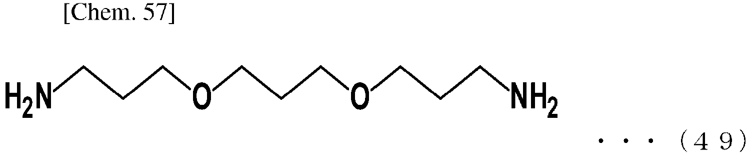

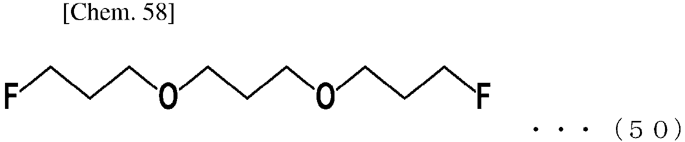

In a nitrogen atmosphere, 1,4-butanediol bis(3-aminopropyl) ether (102.2 mg, 0.50 mmol), 1,1,2,2,3,3-hexafluoropropane-1,3-disulfonylfluoride (632.3 mg, 2.0 mmol), and tetrabutylammonium difluorotriphenylsilicate (TBAT) (270.0 mg, 0.50 mmol) were added to toluene (10 mL), and heating was carried out to 110°C. Diazabicycloundecene (DBU) (304.5 mg, 2.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography (hexane/ethyl acetate = 50/1 to hexane/ethyl acetate = 1/1), and the solvent was distilled off under reduced pressure to obtain an alkyl fluoride compound represented by Formula (50) in a yield of 36%.

Then, by a ¹⁹F-NMR analysis, it was confirmed that the obtained product is the alkyl fluoride compound represented by Formula (50).

¹⁹F-NMR (heavy chloroform) analysis: δ of -221.68 to -222.07 (m, 2F).

### [Example 23 of present invention]

A primary alkylamine compound represented by Formula (51) was used to synthesize an alkyl fluoride compound represented by Formula (52).

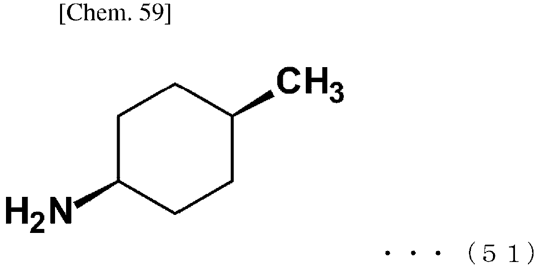

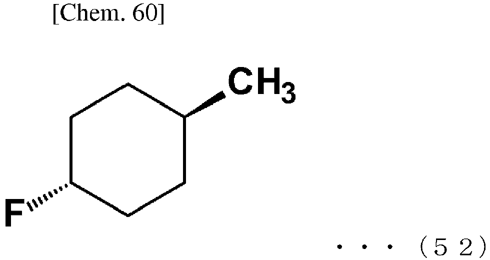

In a nitrogen atmosphere, cis-4-methylcyclohexylamine (56.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to benzene (10 mL), and heating was carried out to 80°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (52) was 7%.

Then, by a ¹⁹F-NMR analysis, it was confirmed that the obtained product is the alkyl fluoride compound represented by Formula (52).

¹⁹F-NMR (heavy chloroform) analysis: δ of -169.48 to -169.80 (m, 1F).

### [Example 24 of present invention]

A primary alkylamine compound represented by Formula (53) was used to synthesize an alkyl fluoride compound represented by Formula (54).

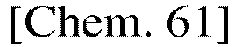

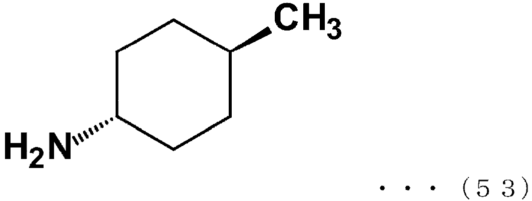

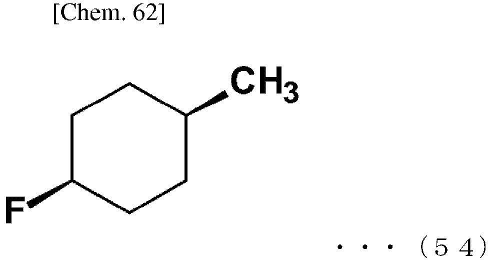

In a nitrogen atmosphere, trans-4-methylcyclohexylamine (56.6 mg, 0.50 mmol), 1,1,2,2,3,3-hexafluoropropane-1,3-disulfonylfluoride (316.2 mg, 1.0 mmol), and tetrabutylammonium difluorotriphenylsilicate (TBAT) (135.0 mg, 0.25 mmol) were added to benzene (10 mL), and heating was carried out to 80°C. Diazabicycloundecene (DBU) (152.2 mg, 1.0 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then chloroform was distilled off under reduced pressure. Benzotrifluoride was added to the obtained crude product as an internal standard to determine the yield in terms of NMR. The yield of the compound represented by Formula (54) was 20%.

Then, by a ¹⁹F-NMR analysis, it was confirmed that the obtained product is the alkyl fluoride compound represented by Formula (54).

¹⁹F-NMR (heavy chloroform) analysis: δ of -183.14 to -183.74 (m, 1F).

### [Example 25 of present invention]

A primary alkylamine compound represented by Formula (55) was used to synthesize an alkyl fluoride compound represented by Formula (56).

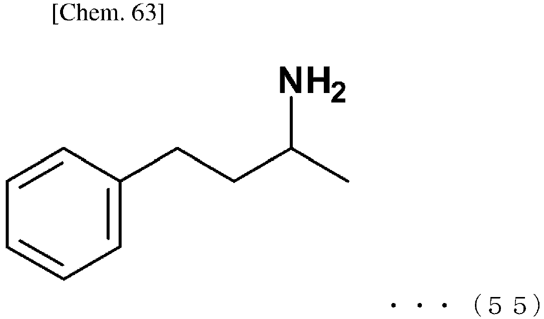

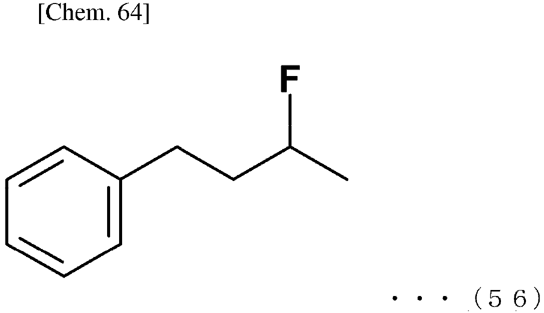

In a nitrogen atmosphere, 3-amino-1-phenylbutane (74.6 mg, 0.50 mmol) and 1,1,2,2,3,3-hexafluoropropane-1,3-disulfonylfluoride (316.2 mg, 1.0 mmol) were added to toluene (5.0 mL), and heating was carried out to 110°C. Diazabicycloundecene (DBU) (152.2 mg, 1.0 mmol) was slowly added thereto, stirring was carried out for 1 hour, and then chloroform (10 mL) and benzotrifluoride as an internal standard were added thereto to determine the yield in terms of NMR. The yield of the alkyl fluoride compound represented by Formula (56) was 35%.

### [Example 26 of present invention]

The above-described primary alkylamine compound represented by Formula (55) described above was used to synthesize the above-described alkyl fluoride compound represented by Formula (56) described above.

In a nitrogen atmosphere, 3-amino-1-phenylbutane (74.6 mg, 0.50 mmol), 1,1,2,2-tetrafluoroethane-1,2-disulfonylfluoride (266.1 mg, 1.0 mmol), and tetrabutylammonium difluorotriphenylsilicate (TBAT) (135.0 mg, 0.25 mmol) were added to toluene (5.0 mL), and heating was carried out to 110°C. Diazabicycloundecene (DBU) (152.2 mg, 1.0 mmol) was slowly added thereto, stirring was carried out for 1 hour, and then chloroform (10 mL) and benzotrifluoride as an internal standard were added thereto to determine the yield in terms of NMR. The yield of the alkyl fluoride compound represented by Formula (56) was 31%.

### [Example 27 of present invention]

The above-described primary alkylamine compound represented by Formula (55) described above was used to synthesize the above-described alkyl fluoride compound represented by Formula (56) described above.

In a nitrogen atmosphere, 3-amino-1-phenylbutane (74.6 mg, 0.50 mmol) and 2,2,3,3,4,4-hexafluoro-4-(fluorosulfonyl)butanoylfluoride (280.1 mg, 1.0 mmol) were added to toluene (5.0 mL), and heating was carried out to 110°C. Diazabicycloundecene (DBU) (152.2 mg, 1.0 mmol) was slowly added thereto, stirring was carried out for 1 hour, and then chloroform (10 mL) and benzotrifluoride as an internal standard were added thereto to determine the yield in terms of NMR. The yield of the alkyl fluoride compound represented by Formula (56) was 3%.

### [Example 28 of present invention]

The primary alkylamine represented by Formula (55) described above was used to isolate a reactant represented by Formula (57) obtained by reacting the primary alkylamine represented by Formula (55) with 1,1,2,2,3,3-hexafluoropropane-1,3-disulfonylfluoride as a deaminative fluorinating agent, and then the alkyl fluoride compound represented by Formula (56) was synthesized.

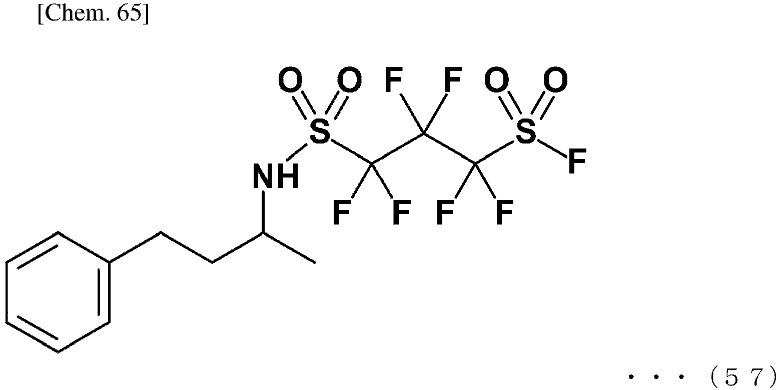

In nitrogen atmosphere, 3-amino-1-phenylbutane (149.2 mg, 1.0 mmol) and 1,1,2,2,3,3-hexafluoropropane-1,3-disulfonylfluoride (379.4 mg, 1.2 mmol) were added to diisopropyl ether (20 mL). Triethylamine (151.8 mg, 1.5 mmol) was slowly added thereto, and stirring was carried out for 27 hours, followed by quenching with a 2 M hydrochloric acid aqueous solution (20 mL) to obtain a reactant.

Chloroform (20 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (20 mL). The organic layer after washing was dried with magnesium sulfate, and then the solvent was distilled off under reduced pressure. The obtained crude product was purified by silica gel column chromatography (hexane to hexane/ethyl acetate = 20/1), and the solvent was distilled off under reduced pressure to obtain a reactant represented by Formula (57) in a yield of 36%.

Then, by a ¹⁹F-NMR analysis, it was confirmed that the reactant represented by Formula (57) has been obtained.

¹⁹F-NMR (heavy chloroform) analysis: δ of -46.76 to -46.69 (m, 1F), -107.22 to -107.35 (m, 2F), -112.40 to -112.63 (m, 2F), -118.38 to -118.49 (m, 2F).

Subsequently, in nitrogen atmosphere, the reactant (160.3 mg, 0.36 mmol) represented by Formula (51) and TBAT (97.2 mg, 0.18 mmol) were added to toluene (7.2 mL). DBU (54.8 mg, 0.18 mmol) was slowly added thereto, and stirring was carried out for 1 hour, followed by quenching with a 2 M hydrochloric acid aqueous solution (10 mL) to obtain a reaction solution.

Chloroform (10 mL) was added to the obtained reaction solution to carry out liquid separation. Then, the organic layer was washed once with a 2 M hydrochloric acid aqueous solution (10 mL), and the obtained organic layer was washed twice with a saturated sodium carbonate aqueous solution (10 mL). The organic layer after washing was dried with magnesium sulfate, and then the solvent was distilled off under reduced pressure. The obtained crude product was purified by silica gel column chromatography, and the solvent was distilled off under reduced pressure to obtain an alkyl fluoride compound represented by Formula (56) in a yield of 68%.

### [Comparative Example 1]

In a case where the primary alkylamine compound represented by Formula (55) described above was used to carry out a reaction without adding a deaminative fluorinating agent, the alkyl fluoride compound represented by Formula (56) was not generated.

In a nitrogen atmosphere, 3-amino-1-phenylbutane (74.6 mg, 0.50 mmol) and tetrabutylammonium difluorotriphenylsilicate (TBAT) (135.0 mg, 0.25 mmol) were added to toluene (5.0 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### [Comparative Example 2]

In a case where the primary alkylamine compound represented by Formula (55) described above was used to carry out a reaction by adding (diethylamino)sulfur trifluoride (DAST) instead of the deaminative fluorinating agent, the generation of the alkyl fluoride compound represented by Formula (56) described above could not be confirmed.

In a nitrogen atmosphere, 3-amino-1-phenylbutane (74.6 mg, 0.50 mmol) and (diethylamino)sulfur trifluoride (DAST) (161.2 mg, 1.0 mmol) were added to toluene (5 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### [Comparative Example 3]

In a case where the primary alkylamine compound represented by Formula (55) described above was used to carry out a reaction by adding nonafluoro-1-butane sulfonylfluoride instead of the deaminative fluorinating agent, the generation of the alkyl fluoride compound represented by Formula (56) described above could not be confirmed.

In a nitrogen atmosphere, 3-amino-1-phenylbutane (74.6 mg, 0.50 mmol) and nonafluoro-1-butane sulfonylfluoride (302.1 mg, 1.0 mmol) were added to toluene (5 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### [Comparative Example 4]

In a case where the primary alkylamine compound represented by Formula (55) described above was used to carry out a reaction by adding propane-1,3-disulfonylfluoride instead of the deaminative fluorinating agent, the generation of the alkyl fluoride compound represented by Formula (56) described above could not be confirmed.

In a nitrogen atmosphere, 3-amino-1-phenylbutane (74.6 mg, 0.50 mmol), propane-1,3-disulfonylfluoride (208.0 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### [Comparative Example 5]

In a case where the primary alkylamine compound represented by Formula (55) described above was used to carry out a reaction by adding 1,2-benzenedisulfonylfluoride instead of the deaminative fluorinating agent, the generation of the alkyl fluoride compound represented by Formula (56) described above could not be confirmed.

In a nitrogen atmosphere, 3-amino-1-phenylbutane (74.6 mg, 0.50 mmol), 1,2-benzenedisulfonylfluoride (242.2 mg, 1.0 mmol), and tetrabutylammonium difluorotriphenylsilicate (TBAT) (135.0 mg, 0.25 mmol) were added to toluene (10 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### [Comparative Example 6]

In a case where the primary alkylamine compound represented by Formula (55) described above was used to carry out a reaction by adding propane-1,3-disulfonylchloride instead of the deaminative fluorinating agent, the generation of the alkyl fluoride compound represented by Formula (56) described above could not be confirmed.

In a nitrogen atmosphere, 3-amino-1-phenylbutane (74.6 mg, 0.50 mmol), propane-1,3-disulfonylchloride (240.9 mg, 1.0 mmol), and tetrabutylammonium difluorotriphenylsilicate (TBAT) (269.9 mg, 0.50 mmol) were added to toluene (10 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### [Comparative Example 7]

In a case where the primary alkylamine compound represented by Formula (55) described above was used to carry out a reaction by adding 1,2-benzenedisulfonylchloride instead of the deaminative fluorinating agent, the generation of the alkyl fluoride compound represented by Formula (56) described above could not be confirmed.

In a nitrogen atmosphere, 3-amino-1-phenylbutane (74.6 mg, 0.50 mmol), 1,2-benzenedisulfonylchloride (275.1 mg, 1.0 mmol), and tetrabutylammonium difluorotriphenylsilicate (TBAT) (269.9 mg, 0.50 mmol) were added to toluene (10 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### [Comparative Example 8]

In a case where the primary alkylamine compound represented by Formula (55) described above was used to carry out a reaction by adding trifluoromethanesulfonic acid anhydride instead of the deaminative fluorinating agent, the generation of the alkyl fluoride compound represented by Formula (56) described above could not be confirmed.

In a nitrogen atmosphere, 3-amino-1-phenylbutane (74.6 mg, 0.50 mmol), trifluoromethanesulfonic acid anhydride (564.3 mg, 2.0 mmol), and tetrabutylammonium difluorotriphenylsilicate (TBAT) (134.9 mg, 0.25 mmol) were added to toluene (10 mL), and heating was carried out to 110°C. Diazabicycloundecene (DBU) (152.2 mg, 1.0 mmol) was slowly added thereto, stirring was carried out for 1 hour, and then benzotrifluoride as an internal standard was added thereto to determine the yield in terms of NMR. As a result, the generation of the alkyl fluoride compound represented by Formula (56) could not be confirmed.

### INDUSTRIAL APPLICABILITY

According to the method for producing an alkyl fluoride compound according to one embodiment of the present invention and the method for producing an optically active alkyl fluoride compound according to one embodiment of the present invention, by using an alkyl fluoride compound having two reaction points in the same molecule as a deaminative fluorinating agent, it is possible to provide an alkyl fluoride compound that is useful as a pharmaceutical, agricultural chemicals, functional materials, or organic synthetic intermediates by fluorination of an amino group, by using, as a raw material for synthesis, the primary alkylamine that is abundant in nature. As a result, the method for producing an alkyl fluoride compound according to one embodiment of the present invention and the method for producing an optically active alkyl fluoride compound according to one embodiment of the present invention have industrial applicability.

## Claims

1. A method for producing an alkyl fluoride compound, the method being **characterized in that** an alkylamine compound represented by General Formula (1), which has at least one amino group bonded to an alkyl carbon atom, is reacted with a deaminative fluorinating agent to generate an alkyl fluoride compound represented by General Formula (2), 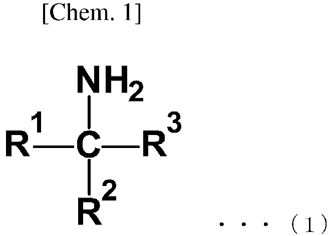
(in General Formula (1), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, R¹, R², and R³ may be bonded to each other to form a 3- to 8-membered carbon ring or a 3- to 8-membered heterocyclic ring having one or more hetero atoms such as oxygen, nitrogen, or sulfur in a ring, and in addition, an amino group (-NH₂) may form a salt by being combined with an acid such as a hydrogen halide or sulfuric acid) 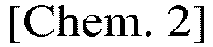 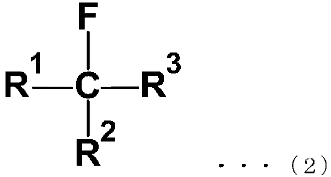
(in General Formula (2), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring).

2. The method for producing an alkyl fluoride compound according to Claim 1, wherein the method is **characterized in that** the deaminative fluorinating agent is a compound represented by General Formula (3), 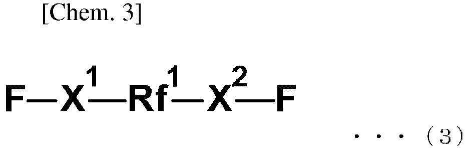 (in General Formula (3), Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or is an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group).

3. A method for producing an alkyl fluoride compound, the method being characterized comprising:
a first reaction step of reacting the alkylamine compound used in the method for producing an alkyl fluoride compound according to Claim 1 with the deaminative fluorinating agent used in the method for producing an alkyl fluoride compound according to Claim 2 to generate an intermediate compound represented by General Formula (4); and
a second reaction step of decomposing the intermediate compound to generate the alkyl fluoride compound in the method for producing an alkyl fluoride compound according to Claim 1, 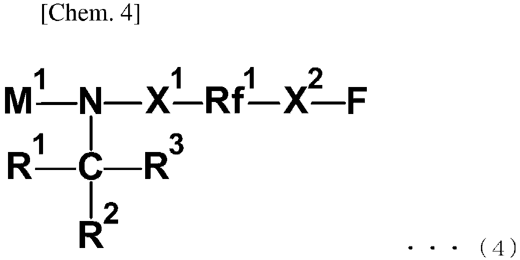
(in General Formula (4), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, R¹, R², and R³ may be bonded to each other to form a 3- to 8-membered carbon ring or a 3- to 8-membered heterocyclic ring having one or more hetero atoms such as oxygen, nitrogen, or sulfur in a ring, and Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group, and M¹ represents hydrogen, a metal, or an organic quaternary ammonium).

4. The method for producing an alkyl fluoride compound according to Claim 3, wherein the method is **characterized in that** the first reaction step and the second reaction step are allowed to proceed at the same time by reacting the alkylamine compound with the deaminative fluorinating agent in a presence of a basic substance.

5. The method for producing an alkyl fluoride compound according to Claim 4, wherein the method is **characterized in that** a nucleophilic fluorine source material is added together with the alkylamine compound, the deaminative fluorinating agent, and the basic substance.

6. A method for producing an alkyl fluoride compound, the method being **characterized in that** a compound represented by General Formula (4) is reacted with a deaminative fluorinating agent to generate an alkyl fluoride compound represented by General Formula (2), 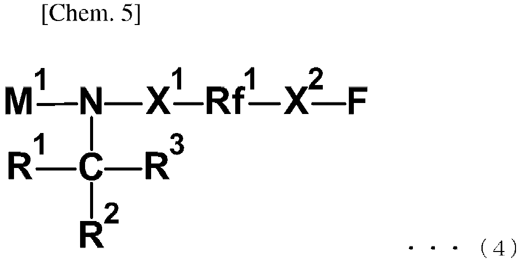
(in General Formula (4), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, R¹, R², and R³ may be bonded to each other to form a 3- to 8-membered carbon ring or a 3- to 8-membered heterocyclic ring having one or more hetero atoms such as oxygen, nitrogen, or sulfur in a ring, and Rf¹ is an alkyl group allowed to have a linear, branched, or cyclic structure having 1 to 10 carbon atoms, which may have 1 to 5 oxygen atoms having bondability to an ether or 1 to 5 unsaturated bonds between carbon atoms, or an aryl group having 6 to 14 carbon atoms, which may have a substituent, where Rf¹ represents a fluoroalkyl group obtained by substituting one or more hydrogen atoms on a carbon atom with fluorine, and X¹ and X² each independently represent a carbonyl group or a sulfonyl group, and M¹ represents hydrogen, a metal, or an organic quaternary ammonium). 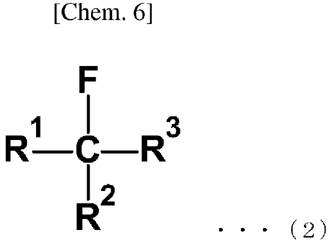
(in General Formula (2), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring).

7. A method for producing an optically active alkyl fluoride compound, the method being **characterized in that** an optically active alkylamine compound represented by General Formula (5), which has at least one amino group bonded to an alkyl carbon atom, is reacted with a deaminative fluorinating agent to generate an optically active alkyl fluoride compound represented by General Formula (6). 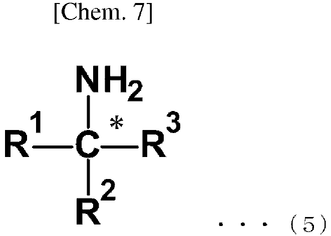
(in General Formula (5), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, R¹, R², and R³ may be bonded to each other to form a 3- to 8-membered carbon ring or a 3- to 8-membered heterocyclic ring having one or more hetero atoms such as oxygen, nitrogen, or sulfur in a ring, and in addition, an amino group (-NH₂) may form a salt by being combined with an acid such as a hydrogen halide or sulfuric acid, and C* represents an asymmetric carbon atom), 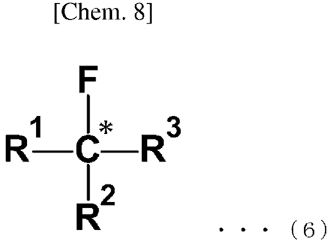
(in General Formula (6), each of R¹, R², and R³ independently represents: a hydrogen atom; an alkyl group; an aryl group; or a carbonyl group which has an alkyl group or aryl group, the alkyl group and the aryl group being allowed to have a linear, branched, or cyclic structure having 1 to 20 carbon atoms and to have a substituent or an unsaturated bond, the alkyl group or the aryl group of the carbonyl group being allowed to have a hydrogen atom, a hydroxy group, a linear, branched, or cyclic structure having 1 to 20 carbon atoms and to have a substituent or an unsaturated bond, and R¹, R², and R³ may be bonded to each other to form a 3- to 8-membered carbon ring or a 3- to 8-membered heterocyclic ring having one or more hetero atoms such as oxygen, nitrogen, or sulfur in a ring, and C* represents an asymmetric carbon atom).
